(19) **Europäisches Patentamt / European Patent Office / Office européen des brevets**

(11) **EP 4 464 681 A1**

(12) # EUROPEAN PATENT APPLICATION

(43) Date of publication:
**20.11.2024 Bulletin 2024/47**

(21) Application number: **24175893.7**

(22) Date of filing: **15.05.2024**

(51) International Patent Classification (IPC):
**C04B 35/18** (2006.01)    **A61N 5/06** (2006.01)
**C04B 35/626** (2006.01)    **C04B 35/64** (2006.01)

(52) Cooperative Patent Classification (CPC):
**C04B 35/18; A61N 5/0625; C04B 35/6263;
C04B 35/64;** A61N 2005/0659; A61N 2005/066;
C04B 2235/3201; C04B 2235/3206;
C04B 2235/3208; C04B 2235/3217;
C04B 2235/3232; C04B 2235/3241;
C04B 2235/3244; C04B 2235/327;
C04B 2235/3284;                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.05.2023 US 202318199660**

(71) Applicant: **Wey, Albert Chin-Tang
Westmont, IL 60559 (US)**

(72) Inventor: **Wey, Albert Chin-Tang
Westmont, IL 60559 (US)**

(74) Representative: **Goddar, Heinz J.
Boehmert & Boehmert
Anwaltspartnerschaft mbB
Pettenkoferstrasse 22
80336 München (DE)**

(54) **HIGH EMISSIVITY CERAMIC COMPOSITE FOR NONTHERMAL FAR INFRARED RADIATION**

(57)    This invention relates to a ceramic composite that may be used for various purposes including for assembly into a therapeutic device for treating a human or animal body with irradiation of nonthermal far infrared (FIR) radiation. More specifically, while all FIR-related prior art is only radiating broadband blackbody thermal radiation, said ceramic composite can emit not only the usual blackbody thermal radiation but also the inventive nonthermal FIR-photon radiation in the 3 - 16 $\mu$m wavelength spectrum. As a result, the overall measurable radiation in 8 - 14 $\mu$m wavelength range from said ceramic composite has an approximated blackbody temperature that is at least 1 °C higher than the actual temperature of said ceramic composite, signifying an effective emissivity greater than 1.0, while 1.0 emissivity is a theoretical limit assigned to an ideal black body and thus unsurpassable. It is an outcome of adding the 3 - 16 $\mu$m band of nonthermal FIR-photon radiation to the continuous 4 - 1,000 $\mu$m band of blackbody thermal radiation.

FIG. 6

EP 4 464 681 A1

(52) Cooperative Patent Classification (CPC): (Cont.)
C04B 2235/3418; C04B 2235/5445;
C04B 2235/6562; C04B 2235/6567;
C04B 2235/76

**Description**

**CROSS REFERENCE TO RELATED APPLICATIONS**

This application is a continuation-in-part application claiming the benefit of application number 17/473,799 filed September 13, 2021. This application is incorporated by reference herein in its entirety.

**BACKGROUND**

Field of Invention

This invention relates to a ceramic composite that may be used for various purposes including for assembly into a therapeutic device for treating a human or animal body with irradiation of nonthermal far infrared (FIR) radiation. More specifically, while all FIR-related prior art is only radiating broadband blackbody thermal radiation, said ceramic composite can emit not only the usual blackbody thermal radiation but also the inventive nonthermal FIR-photon radiation in the 3 - 16 $\mu$m wavelength spectrum. As a result, the overall measurable radiation in 8 - 14 $\mu$m wavelength range from said ceramic composite has an approximated blackbody temperature that is at least 1 °C higher than the actual temperature of said ceramic composite, signifying an effective emissivity greater than 1.0, while 1.0 emissivity is a theoretical limit assigned to an ideal black body and thus unsurpassable. It is an outcome of adding the 3 - 16 $\mu$m band of nonthermal FIR-photon radiation to the continuous 4 - 1,000 $\mu$m band of blackbody thermal radiation.

Description of Prior Art

Blackbody radiation is the thermal electromagnetic radiation emitted by a black body that is in thermodynamic equilibrium with its environment. It has a specific, continuous spectrum of wavelengths that depend only on the body's temperature. The emitted radiation is closely described by Planck's law and because of its dependence on temperature, Planck radiation is said to be thermal radiation. Quantum theoretical explanation of Planck's law perceives the radiation as a gas of massless, uncharged, bosonic particles, namely photons. Photons are viewed as the carriers of the electromagnetic interaction between electrically charged elementary particles.

The thermal radiation spontaneously and continuously emitted by many ordinary objects can be approximated as black-body radiation. A black body at room temperature (25 °C, or 298 °K) radiates mostly in the infrared spectrum, spanning across 4 - 1,000 $\mu$m spectrum with a peak at 9.4 $\mu$m wavelength and 90% of total energy amassed in the 6 - 25 $\mu$m wavelength range.

This becomes an erroneous belief that prior art is entrenched in. They have mistakenly used this blackbody thermal radiation in 6 - 25 $\mu$m wavelength spectrum and wished for an effect that only the nonthermal FIR-photon radiation in the 3 - 16 $\mu$m wavelength range would offer.

For example, biochemical reaction rates may be increased respectively to a different degree by blackbody thermal radiation or by specific FIR-photon radiation at 3 - 16 $\mu$m wavelengths. The chemical reaction rate is described by the Arrhenius equation:

$$k = A \ exp \ (\text{-} \ E_A \, / \, RT)$$

where $k$ is the rate constant, $A$ = pre-exponential factor,
$E_A$ = activation energy required (or called "activation barrier"),
$R$ = gas constant, and $T$ = temperature in °K.

Absorption of blackbody thermal radiation can increase the body's temperature. As the temperature ($T$) increases, the rate of reaction (k) also increases. At a rough approximation for many reactions happening at around room temperature, the rate of reaction doubles for every 10 °C (or 10 °K) rise in temperature. For a typical FIR-thermal therapy with thermal radiation from FIR-emitting materials of prior art, it may increase the skin temperature by about 3 - 5 °C, resulting in only about 30% increase in reaction rate.

In contrast, FIR photons at 3 - 16 $\mu$m wavelengths can be absorbed by molecules in the body causing molecular vibrations. It has an effect on reducing the activation barrier ($E_A$) by about 8-40 KJ, given by the formula: $E_\lambda$ (KJ) = 120 KJ/ $\lambda(\mu$m). Correspondingly, the reaction rate may be improved exponentially, $exp \ (E_\lambda \, / \, RT)$, by as much as 5,000 - 10,000%.

This phenomenal effect is indeed the motivating force of present invention, proposing to develop a ceramic composite that may radiate not only the conventional blackbody thermal radiation as described by prior art, but also the unprecedented, additional nonthermal FIR-photon radiation in the 3 - 16 $\mu$m wavelength spectrum.

Commonly, prior art has misused blackbody thermal radiation, which is evidenced by the fact that all prior art only searches for a FIR material that has an emissivity $\varepsilon$ as close to the theoretical limit ($\varepsilon = 1.0$) as possible.

All physical substances in solid, liquid, or gaseous states can emit energy via a process of electromagnetic radiation because of vibrational and rotational movement of their molecules and atoms. Planck radiation is the greatest amount of radiation that any body at thermal equilibrium can emit from its surface, whatever its chemical composition or surface structure may be.

The emissivity of the surface of a material is its effectiveness in emitting energy as thermal radiation. Quantitatively, emissivity ($\varepsilon$) is the ratio of the radiant energy emitted by a surface to that emitted by a black body at the same temperature, given by the Stefan-Boltzmann law. The ratio varies from 0 to 1 ($0 < \varepsilon < 1$). All real objects have an emissivity less than 1.0, and emit radiation at correspondingly lower rates. For examples, daily objects like fabric, glass, charcoal, concrete, porcelain, rubber, and sands, all have an emissivity between 0.80 and 0.98.

Since FIR-radiation became a hopeful alternative therapy, numerous inventions have geared towards the development of a highly effective FIR-emitting material with a desired emissivity greater than 0.9 in mind (i.e., $0.9 < \varepsilon < 1.0$), while 1.0-emissivity is well regarded as an unsurpassable hypothetical limit.

The raw metal oxides used by prior art for making FIR composites usually have an individual emissivity between 0.5 and 0.9, depending on the degree of oxidation and surface preparation. The most commonly used FIR-emitting oxides include oxides of aluminum, silicon, zirconium, titanium, magnesium, chromium, iron, cobalt, copper, calcium, sodium, and so on. All materials are processed to produce a specific shape, hoping to refine the FIR radiation rate (emissivity) through sintering.

As described above, the key aspect in the design of a conventional FIR-emitting composite was to have an emissivity as close to 1.0 as possible. Nevertheless, all FIR composites of prior art resulted in only an emissivity around 0.85 - 0.95, because they had treated all constituent oxides aimlessly and indifferently (for example, U.S. Pat No. 8,285,391, 9,308,388, and 9,962,441, each of which is incorporated by reference in their entireties). None of prior art targeted emissivity higher than 1.0, as it was regarded as a theoretical limit assigned only to an ideal black body and thus unreachable.

The present inventor previously shared a similar view, which was reflected by the making of a FIR-emitting ceramic composite that required heating when higher radiation was needed (for examples, U.S. Pat No. 10,610,699, and U.S. Pat Appl. No. 20210228903, both of which is incorporated by reference in their entireties). However, it was not until the present inventor later researched the characteristics of transition metal oxides (TMO) and discovered that by tuning the band gap and lattice constant of the final crystal structure, a high emissivity in the 3 - 16 $\mu$m wavelength spectrum would become possible (U.S. Pat. Appl. No. 17/473,799, which is incorporated by reference in their entireties). Moreover, after intensive research based on the finding, the present inventor further realized a specific ceramic composite that could emit FIR-photons at 3 - 16 $\mu$m wavelengths much more efficiently and reliably, as described herein.

The ceramic composite of present invention comprises a solid solution. A solid solution is a uniform mixture of two or more crystal solids that share a common crystalline lattice. Solvent is the element or compound that is present in the greatest amount (> 50 wt.%), while solute denotes an element or compound present in a minor concentration (< 50 wt.%). The solute may incorporate into the solvent crystalline lattice either substitutionally by replacing a solvent particle in the lattice, or interstitially by fitting into the space between solvent particles. Both of these types of solid solution affect the properties of the material by distorting the crystalline lattice and disrupting the physical and electrical homogeneity of the solvent material.

Additionally, the Hume-Rothery rules define the conditions under which an element in solute could dissolve in a solvent, forming a solid solution. Along with the rules, solute (or impurity atoms) may substitute for the atoms in solvent, if the solute and solvent have similar atomic radii (15% or less difference), electronegativities, valency, and same crystal structure.

In present invention, a solid solution is expected to consist of aluminum oxide ($Al_2O_3$) as part of solvent. Because, aluminum oxide provides a good source for building the necessitated octahedral crystalline framework in the host lattice for accommodating the "persistent FIR-photon emission mechanism" proposed by the present invention. Intrinsically, adding silicon oxide ($SiO_2$) to aluminum oxide as solvent may help construct a more stable aluminosilicate oxoanions ($AlSiO_4^-$) infrastructure for the crystal field and thus becomes a meaningful option.

Obeying the Hume-Rothery rules, iron (Fe) and chromium (Cr) come to be the perfect candidates as solute for substituting aluminum (Al) in the host lattice. The oxides of these elements ($Al_2O_3$, $Cr_2O_3$, $Fe_2O_3$) all have similar valency and same octahedral crystalline structure. All elements have same electronegativities (III): $Al^{3+}$, $Cr^{3+}$, and $Fe^{3+}$. Besides, the ionic radii of $Cr^{3+}$ (7.55 Å) and low-spin $Fe^{3+}$ (6.9 Å) are within 15% difference limit of the radius of $Al^{3+}$ (6.8 Å).

Among said features, the use of transition metal (TM) in an octahedral crystalline structure plays a crucial role in the planned persistent FIR-photon emission mechanism. The elements iron and chromium both belong to Groups 3 - 12, transition metal elements in the Periodic Table. Precisely, the TM-octahedron structure represents an important building block in the ceramic composite of present invention.

Transition Metal Oxides (TMOs) constitute probably one of the most interesting classes of solids, exhibiting a variety of structures and properties. The nature of metal-oxygen (M-O) bonding within TMOs can vary between nearly ionic to highly covalent or metallic. The unusual properties of TMOs are clearly due to the unique nature of the outer *d*-electrons in the five degenerate orbits.

Based on Molecular Orbital (MO) theory of transition metal complexes, the characteristics of transition metal-ligand (M-L) bonds become well-defined by analyzing the molecular orbitals of a *3d* metal (M) coordinated by six identical ligands (L) in an octahedral complex [$ML_6$]. As a result of the interaction between the metal *d* and ligand orbitals, bonding, non-bonding and anti-bonding complex molecular orbitals are formed. Normally, the energy levels of the ligand orbitals are lower than those of the metal orbitals. The bonding orbitals have more ligand character, and non-bonding and anti-bonding orbitals have more metal character.

Concepts from MO theory are valuable in understanding the reactivity of coordination compounds. One of the basic ways of applying MO concepts to coordination chemistry is in Ligand Field Theory (LFT). LFT looks at the effect of donor (ligand) atoms on the energy of *d* orbitals in the metal complex. LFT can be considered as an extension of Crystal Field Theory such that all levels of covalent interactions can be incorporated into the model. Treatment of the bonding in LFT is generally done using MO theory.

Crystal Field Theory (CFT) is a bonding model that describes the electronic structure of transition metal (TM) complexes. CFT focuses on the interaction of the five valence (n-1)*d* orbitals with a "field" of negative charge (electrons) from ligands arranged around a TM ion. CFT was developed to explain the interesting spectroscopic properties of TM complexes.

Suppose a TM complex has an octahedral coordination sphere and assume the six ligands all lie along the x, y and z axes. There are two *d* orbitals that will interact very strongly with these ligands: the $d_{x^2-y^2}$, which lies directly on the x- and y-axis, and the $d_{z^2}$, which lies directly on the z-axis. These two orbitals will be raised relatively high in energy due to the bonding interactions with the donor (ligand) orbitals. These orbitals are sometimes called the "$e_g$" set of orbitals. The term "$e_g$" comes from the mathematics of symmetry.

On the other hand, the other three *d* orbitals, $d_{xy}$, $d_{xz}$ and $d_{yz}$, all lie between the bond axes with the donor ligands, rather than banging them directly. These orbitals will interact less strongly with the donor electrons. These orbitals are sometimes called the "$t_{2g}$" set of orbitals.

An octahedral arrangement of six negative charges (ligands) around a TM ion causes the five *d* orbitals to split into two sets ($e_g$ and $t_{2g}$) with different energies. This is called Crystal Field Splitting. Crystal field splitting does not change the total energy of the *d* orbitals, but it can change the energy of the electrons.

In a crystalline environment, the fivefold degeneracy of *d*-orbitals is partially lifted by the electrostatic crystal field and by hybridization with the ligand ions. According to CFT model, factors that affect orbitals energies may include: the nearest neighbor oxygens, electrostatic potential from the rest of crystal, kinetic energy from the hybridization of the metal orbitals with the nearest neighbor ligands, radial relaxation of the 3*d* orbitals, the Coulomb and exchange contributions within the 3*d* shell, and the excitations from the other orbitals (screening effects).

The orbital degeneracy of $e_g$ and $t_{2g}$ sets of orbitals may further be lifted by the coupling to the lattice, which is called the Jahn-Teller effect. It often occurs when a group of octahedral crystal structures are processed to build, for example, a tetragonal unit cell that causes tetragonal distortion in the octahedral complexes.

Typically, the unit cells in a polycrystal are not unique. As the ceramic composite of present invention may contain randomly oriented crystalline regions, the unit cells may be processed to be any of the seven crystal systems, including triclinic, monoclinic, orthorhombic, tetragonal, trigonal, hexagonal, and cubic, that strongly depends on the processing parameters.

Jahn-Teller effect is observed for TM-ions with electronically degenerate states. In the electronically degenerate state, the orbitals are said to be asymmetrically occupied and hence get more energy. Therefore, the system tries to get rid of extra energy by lowering the overall symmetry of the molecule via undergoing distortion. It is known as Jahn-Teller distortion effect.

In other words, the orbital degeneracy can be removed by either elongating the bonds along the z-axis ("Z-out" distortion), or shortening the bonds along the z-axis ("Z-in" distortion). In this arrangement, the octahedrally symmetrical molecules may be distorted to a different degree, from a tetragonal geometry ($a = b \neq c$, $\alpha = \beta = \gamma = 90°$) to a triclinic geometry ($a \neq b \neq c$, $\alpha \neq \beta \neq y$). Throughout this patent application, a tetragonal geometry is used as exemplar due to its simplicity in CFT modeling. The case may be extended to any other crystal system.

The Jahn-Teller (J-T) distortion effects may be briefly categorized as follows:
Z-out J-T distortion: The energies of d-orbitals with z factor ($d_{z^2}$, $d_{xz}$, $d_{yz}$) are lowered since the bonds along the z-axis are elongated. This is the most preferred distortion and occurs in most of the cases, especially when the degeneracy occurs in $e_g$ level.

Z-in J-T distortion: The energies of d-orbitals with z factor are increased since the bonds along the z-axis are shortened. For example, this type of distortion is observed in case of octahedral $d^1$ configuration. The only electron will now occupy the $d_{xy}$ orbital with lower energy than the other two orbitals in $t_{2g}$ group, $d_{xz}$ and $d_{yz}$.

Static J-T distortion: Some molecules show tetragonal shape under all conditions at lower and relatively higher temperatures. It is observed when the degeneracy occurs in $e_g$ orbitals. Hence the distortion is strong and permanent.

Dynamic J-T distortion: In some molecules, the distortion is not seen either due to random movements of bonds or else the distortion is so weak as to be negligible. However, the distortion can be seen by freezing the molecule at lower temperatures.

As highlighted above, the principles behind the present invention involve Solid Solution, TM Coordination Complexes, Octahedral Crystalline Structure, MO Theory, LFT, CFT, Crystal Field Splitting, and J-T Distortion Effects.

Appreciating the featuring sciences, one may start exploring the case of constructing FIR-luminescence centers with octahedral coordination complexes $[CrO_6]^{3+}$, or the like, that are to be implanted in the host lattice of the ceramic composite of present invention, as disclosed below.

Photon emission takes place when the matrix is capable to luminescence and contains luminescence centers. Any luminescent material is characterized by its host lattice and certain luminescent centers. The emission of luminescence light (visible, near-IR, or FIR) occurs as a result of a radiative electronic transition in which an electron jumps from a higher energy state to a lower one, the difference in energy being released as a photon. It is obvious that the electron must first be excited into higher energy states by some means and the system can return to the ground state spontaneously under emission of the stored energy from the excited state as photons.

The exemplar building block of the planned FIR-luminescence centers in present invention is $[CrO_6]^{3+}$, which consists of a chromium-III ion ($Cr^{3+}$) surrounded by six oxygen anions ($O^-$). The $Cr^{3+}$ ion uses 6 orbitals from the *4s, 4p* and *4d* levels of chromium atom to accept lone pairs electrons from the oxygen molecules for bonding with oxygen, forming $[CrO_6]^{3+}$.

For the $Cr^{3+}$ ion in a $[CrO_6]^{3+}$ octahedron, a total of 3 electrons are removed, one from the *$4s^1$* and two from the *$3d^5$*. This results in only 3 electrons remaining in the 3d-orbitals, denoted by *$3d^3$*. The electrons in the 3*d* level of Cr-atom are not involved in the bonding. In this case that the ligands are oriented on the cartesian coordinate axes, the transition metal will still own *3d* orbitals, $d_{xy}$, $d_{xz}$, and $d_{xz}$, which do not interact with the ligands and are considered "nonbonding" orbitals.

As the FIR-luminescence center of present invention is founded on the $Cr^{3+}$ ion with a *$3d^3$* electronic configuration in an octahedral site, the unit cell of the ceramic composite has to be deliberately sintered into a crystal geometry that produces a slight "Z-in J-T distortion". After sintering, the degeneracy of orbitals in tag group is removed. The $d_{xy}$ orbital would have lower energy than the other two, $d_{xz}$ and $d_{yz}$. Correspondingly, a pair of electrons in $Cr^{3+}$ ion will occupy the $d_{xy}$ orbital, while the lone electron occupies one of the $d_{xz}$ and $d_{yz}$ orbitals.

By design, the $Cr^{3+}$ ion would be placed in a weak electrostatic crystal field, formed by the surrounding anions (or oxoanions) and cations. Based on CFT model, the energy $\delta_2$ of the crystal field splitting between $[d_{xy}]$ and $[d_{xz}$ & $d_{yz}]$ orbitals is about 100 - 315 meV, corresponding to a wavelength spectrum in the range of 4 - 12 $\mu$m, given by the formula: Ea, (KJ) = 120 KJ/ $\lambda(\mu m)$.

The CFT model determines the energy of an electronic excitation as a function of lattice deformation. Governed by the Transition Rule (the Selection Rule), the transitions require the quantum overlap of the lattice zero-point vibrations. Likewise, the Franck-Condon principle states that during an electronic transition, a change from one vibrational energy level to another will be more likely to happen if the two vibrational wave functions overlap more significantly. The overlap of two vibrational wave functions is called Franck-Condon Overlap (or Franck-Condon Factor).

The Franck-Condon principle helps to analyze the vibronic transitions and explains the intensity distribution in the vibrational bands. It plays an important role in understanding the nature of optical transitions and the optical spectroscopic processes taking place in the TM-ion sites. The model provides a guidance on how to design a photoluminescence profile that may include the specified 3 - 16 $\mu$m wavelength range of present invention, while the vibronic transitions may actually occur within the theoretical 4 - 12 $\mu$m range.

The transitions of *$3d^3$* electrons between the $t_{2g}$ orbitals of $Cr^{3+}$ ion in an octahedral complex act as an activating center of FIR-luminescence. The energy ($\delta_2$), stored as electrostatic potential in the crystal field splitting, can be liberated by thermal stimulation that results in "stimulated FIR-photon emission" from the luminescence centers.

To sum up, a unique "persistent FIR-photon emission mechanism" proposed by present invention differs very much from the teaching of prior art, and can be described as follows:

The solid solution of present invention comprises at least 10 wt.% aluminum oxide ($Al_2O_3$) in solvent and at least 3 wt.% chromium oxide ($Cr_2O_3$) and/or iron oxide ($Fe_2O_3$) in solute, where wt.% denotes the percentage by total weight of the composite. In practice, chromium oxide and iron oxide may be used jointly or separately as solute.

The combined use of at least 10 wt.% aluminum oxide in solvent and 3 wt.% chromium oxide and/or iron oxide as solute in present invention represents a threshold (minimum requirement) for building enough $[CrO_6]^{3+}$ octahedrons as FIR-luminescence centers for the resultant ceramic composite to be effective in generating and emitting nonthermal FIR-photon radiation. A combination of more such components would be desired for better results.

The solution is to be sintered at a temperature that is in the range of 0.5 to 0.75 of the melting temperature of the solvent. The length of sintering time is determined by the chosen temperature such that a host lattice may be constructed with aluminum ions in octahedral structures, and for the meantime, a part of aluminum ions ($Al^{3+}$) in the octahedral sites may be substituted by chromium ions ($Cr^{3+}$) or iron ions ($Fe^{3+}$) through counter diffusion of cations.

During sintering, the ceramic composite is further deliberately processed to cause a slight "Z-in J-T distortion" within its constituent unit cells, through manipulating phase transformations of the ingredient oxides with a precalculated heating cycle. In this way, the degeneracy of orbitals in $t_{2g}$ group is removed such that the $d_{xy}$ orbital may have lower energy than $d_{xz}$ and $d_{yz}$. For the 3 electrons in $3d^3$ configuration of $Cr^{3+}$ ion, a pair of electrons will occupy the $d_{xy}$ orbital, while the lone electron occupies one of the $d_{xz}$ and $d_{yz}$ orbitals.

When such a ceramic composite is subjected to thermal stimulation, it triggers dynamic J-T distortion. On one extreme of thermal stimulation, the z-axis of $[CrO_6]^{3+}$ octahedron elongates to generate a temporary Z-out J-T distortion. Thus, the $d_{xy}$ orbital swings to have higher energy than $d_{xz}$ & $d_{yz}$. It means that the two electrons in $d_{xy}$ orbital are now in an excited state and have to drop down to the lower energy orbits $d_{xz}$ & $d_{yz}$, by emitting two photons equivalent to the energy $\delta_2$. By design, the wavelengths of photons would fall in the 3 - 16 $\mu$m range. After emission of two photons, all three electrons are then positioning at the lower energy orbitals, $d_{xz}$ & $d_{yz}$.

By the same token, on the other extreme of thermal stimulation, the z-axis of $[CrO_6]^{3+}$ octahedron shortens to trigger a temporary Z-in J-T distortion. The $d_{xy}$ orbital swings to have lower energy than $d_{xz}$ & $d_{yz}$. Again, two of the three electrons present in $d_{xz}$ and $d_{yz}$ orbitals must drop down to the lower orbit $d_{xy}$ by emitting two photons equivalent to the energy $\delta_2$.

If the ceramic composite has been fabricated right as planned, the FIR-photon generation and emission process will continue and last indefinitely with the induced effect of "thermally stimulated dynamic J-T distortions."

Yet, there is still one key factor missing from above description of persistent FIR-photon emission mechanism that links electronic excitations and stimulated photon emissions at the octahedral sites, namely phonons (or the wave of lattice vibrations). As such, the mechanism proposed by present invention may be called "persistent phonons-activated FIR-photon emission".

A phonon is the quantum mechanical description of an elementary vibrational motion in a condensed matter, in which a lattice of atoms or molecules uniformly oscillates at a single frequency. In classical mechanics, this designates a normal mode of vibration. Any arbitrary lattice vibration can be considered to be a superposition of these elementary vibration modes. For a crystal that has at least two atoms in its primitive cell, the dispersion relations exhibit two types of phonons, specifically optical and acoustic modes.

A lattice at any temperature above Absolute Zero (0 °K or -273 °C) has an energy that is not constant, but fluctuates randomly. These energy fluctuations are caused by random lattice vibrations, which can be viewed as a gas of phonons. Because these phonons are generated by the temperature of the lattice, they are sometimes designated thermal phonons.

The thermodynamic properties of a solid are directly related to its phonon structure. The phonon density of states (DOS) describes the phonon dispersion relations of all possible phonons and determines the heat capacity of a crystal. The heat capacity is dominated by the high-frequency part of the distribution (optical modes), while thermal conductivity is the result of the low-frequency region (acoustic modes).

Thermal phonons can be created and destroyed by random energy fluctuations. In statistical mechanics, this means that the chemical potential for adding a phonon is zero. The behavior of thermal phonons is similar to the photon gas produced by an electromagnetic cavity. It turns out that the electromagnetic field acts like a set of harmonic oscillators, that gives rise to Black-body radiation. Both gases of phonons and photons behave similarly in the electromagnetic cavity and obey the Bose-Einstein statistics.

This similarity is surely not coincidental. Planck's law describes the characteristic spectral distribution for electromagnetic radiation in thermodynamic equilibrium. For matter or net energy not enclosed in an electromagnetic cavity (such as an ideal black body), thermal radiation can be approximately explained by Planck's law. In view of quantum mechanics, both phonons and photons are identical and interchangeable in such an electromagnetic cavity.

Planck's law arises as a limit of the Bose-Einstein distribution that describes the energy distribution of non-interactive bosons in thermodynamic equilibrium. In the case of massless bosons such as photons and phonons, the chemical potential is zero and the Bose-Einstein distribution reduces to the Planck distribution.

The Kirchhoffs law of thermal radiation states that in thermodynamic equilibrium, the thermal radiation emitted from an ideal black body would have a unique universal spectral radiance as a function of temperature, independent of the chemical characteristics of its materials. And, the Equation of Radiative Transfer describes the way in which radiation is affected as it travels through a material medium. When the material medium is in thermodynamic equilibrium, the thermal radiation from a black body is always equal to the full amount specified by Planck's law.

As explained by Planck, a black body absorbs all and reflects none of the electromagnetic radiation incident upon it. The reflection and transmission of radiation at the interface obey the Stokes-Helmholtz reciprocity principle. According to the reciprocity principle, radiation from the interior of a black body is not reflected at its surface, but is fully transmitted to its exterior.

Based on abovesaid quantum mechanics principles, one may envision the following:

The absorption of incident thermal radiation (photons) by a black body can generate wave of lattice vibrations (phonons) in the body. It is seen as the thermal radiation (photons) being converted into lattice vibrations (phonons) at the interface, by means of "photon-phonon coupling" with energy and momentum conservations. Likewise, the emission of Planck radiation (thermal radiation) at the surface of a black body can also be seen as the generation and release of photons by the lattice vibrations (phonons) in the body to keep thermodynamic equilibrium, via the reverse process of "photon-phonon coupling".

The absorptivity (i.e., transferring energy from incident photons to phonons in a condensed matter) and the emissivity (transferring energy from phonons back to photons) are each separately properties of the molecules of the material, and they depend differently upon the distributions of the states of molecular excitation on the occasion. Yet, on occasions when the material is in thermodynamic equilibrium, the emissivity and absorptivity become equal. It is so expected that all electromagnetic radiation (thermal radiation) absorbed by a black body will be fully transmitted to its exterior when it is in thermodynamic equilibrium with the surroundings. In fact, Kirchhoff had demonstrated the equality of absorptivity and emissivity for a black body in thermodynamic equilibrium.

The equality of absorptivity and emissivity became a code of belief obeyed by prior art. All prior art observed this prescribed doctrine as a guidance when it came to design FIR-emitting composites and hoped to develop a FIR material that would have an emissivity $\varepsilon$ as close to the theoretical limit ($\varepsilon = 1.0$) as possible. Because, an ideal black body has an impeccable absorptivity equal to 1.0 (i.e., no reflection), so will the emissivity.

Nonetheless, what prior art has ignored is the fact that Planck's law is applicable only when there is no net flow of energy in the body. When there is energy flow present in the body, as in the ceramic composite of present invention, it must involve Einstein coefficients deliberated by Albert Einstein.

The Principle of Detailed Balance requires that each elementary process is equilibrated by its reverse process when at thermodynamic equilibrium. In 1916, Albert Einstein applied this principle on an atomic level to the case of an atom radiating and absorbing radiation due to transitions between two particular energy levels. Einstein gave a deeper insight into the Equation of Radiative Transfer and Kirchhoffs law for this type of radiation.

If level 1 is the lower energy level with energy $E_1$, and level 2 is the upper energy level with energy $E_2$, then the frequency $\nu$ of the radiation radiated or absorbed will be determined by Bohr's frequency condition:

$$E_2 - E_1 = h\nu$$

According to Einstein's model, there are three parameters, $A_{21}$ (Spontaneous emission), $B_{21}$ (Stimulated emission) and $B_{12}$ (Photon-absorption), associated with the photon frequency $\nu$ produced by the transition between two energy levels (states). These parameters are known as the Einstein coefficients, and each line in a spectrum has its own set of associated coefficients.

When in a case that the atoms and the radiation field are in equilibrium, the radiance will be given by Planck's law and, by the Principle of Detailed Balance, the sum of absorption and emission rates must be zero. It implies that the emission coefficient and absorption coefficient are equal. So, for the case of isotropic absorption and emission, the relationships between the Einstein coefficients will yield the expression of Kirchhoffs law expressed before. Indeed, all FIR-related prior art has fallen into this set-up.

However, when there is a net flow of energy in the body, as in the case of present invention with a persistent FIR-emission mechanism, the emission coefficient and absorption coefficient defined in the radiative transfer must be expressed in terms of the Einstein coefficients. These coefficients apply to both atoms and molecules.

In plain English, in the case that represents the prior art, one may imagine a regular material body at a temperature $T_1$, lying in a thermal radiation field at a temperature $T_2$, where $T_2 > T_1$. For the material body having an absorptivity $\alpha$ as the fraction of incident radiation absorbed by the body, that incident energy is absorbed at a rate a. Meanwhile, the body emits its own thermal radiation at temperature $T_1$ and one may define the emissivity $\varepsilon$ of the material body. When there is thermodynamic equilibrium at temperature T, when $T = T_1 = T_2$, the thermal radiation from the body will be equal to the incident radiation absorbed by the body. In other words, the absorptivity is equal to the emissivity, or $\alpha = \varepsilon$, as pointed out by Kirchhoff, and so believed by all prior art.

Conversely in the other case that represents present invention, one needs take into consideration of a net flow of energy in the ceramic composite that is created by the persistent phonons-activated FIR-photon emission mechanism of present invention.

Again, assume the ceramic composite is at a temperature $T_1$, lying in a thermal radiation field at a temperature $T_2$, where $T_2 > T_1$. Said composite absorbs incident thermal radiation (photons) and converts the radiative heat energy into lattice vibrations (phonons) through "photon-phonon coupling". The lattice vibrations instantaneously trigger dynamic J-T distortions in the crystalline lattices of said ceramic composite, which alternatively swing between "Z-in" and "Z-out" configurations in $[CrO_6]^{3+}$ octahedral sites. As a result, FIR photons at 3 - 16 $\mu$m wavelengths are generated and spontaneously emitted. In this case, there exists a nonzero Einstein's coefficient for Stimulated Emission, $B_{21} \neq 0$.

Meanwhile, even right at the very moment that the ceramic composite is in thermodynamic equilibrium with its environment at temperature T, when $T = T_1 = T_2$, lattice vibrations in said composite persist and phonon-activated photon emissions continue. The ceramic composite uninterruptedly emits photons at 3 - 16 $\mu$m wavelengths and thus undergoes loss of energy. It then needs absorbing incident radiation to increase its temperature for keeping up thermodynamic equilibrium. On the other hand, the additional energy absorbed from incident radiation will cause lattice vibrations and trigger the emission of FIR-photons. The round goes on and on, and the FIR-photon emission lasts forever.

Precisely, the technology developed herein by the present inventor is an equivalence to the existing art of Persistent Luminescence, also known as Long-Lasting Phosphors (LLP).

LLP's applications include the uses in night vision, temperature sensors, LEDs, LASERs. medical diagnostics, and in-vivo bio-imaging (e.g., tumor marking). In those applications, the phosphors may be excited by x-ray or UV light and the excitation energy is stored in the distorted octahedral sites of the phosphors. The stored energy may be liberated by thermal, optical or other physical stimulations, resulting in stimulated emissions in visible light or NIR (near-infrared).

The LLPs use the wide band energy $\Delta_o$ (the subscript "o" denotes "octahedral") of the crystal field splitting in an octahedral arrangement between the two sets of orbitals ($e_g$ and $t_{2g}$), in which $\Delta_o > 2$ eV. For example, a blue LED can be made from a chromium-(III) doped barium borate ($BaB_2O_4$:$Cr^{3+}$), with $\Delta_o = 16,746$ cm$^{-1}$ (equivalent to 2.08 eV) that results in the emission of blue light, $\lambda = 596$ nm.

Alternatively, the present invention uses a rather insignificant energy difference between the d-orbitals in $t_{2g}$ group ($d_{xy}$ vs. $d_{xz}$ & $d_{yz}$), with an energy $\delta_2$ in the range of 100 - 315 meV that enables FIR-photon emission in the 3 - 16 $\mu$m wavelength spectrum. In this method, the energy difference ($\delta_2$) is so small that the FIR-photon emission may be activated simply by the natural energy fluctuations associated with lattice vibrations (phonons) of a condensed matter at room temperature. Therefore, there is no need of an external excitation energy source for stimulating the FIR-photon emissions of present invention.

In summary, all FIR-related prior art teaches only the practice of blackbody thermal radiation and adheres to the concept of the equality of absorptivity and emissivity, which is derived specifically for a black body that has no net flow of energy within.

Accordingly, all prior art believes that the thermal radiation emitted from any body is always equal to the full amount of the thermal radiation absorbed, as specified by Planck's law. The practice is reflected by the FIR-materials developed by prior art always having the emissivity of an ideal black body ($\epsilon = 1.0$) as a target, while 1.0 emissivity is deemed a theoretical limit and unsurpassable. Though, prior art constantly falls short and concedes to an emissivity in 0.85 - 0.95.

And, when it comes to calculate the effect of FIR-radiation on therapeutic applications, all prior art aims at "heating" the treated body part. It indicates that the FIR-radiations created by prior art are nothing but thermal radiation. As mentioned before, Planck radiation has a continuous spectrum of wavelengths spanning across 4 - 1,000 $\mu$m at room temperature, with 90% of total energy amassed in the 6 - 25 $\mu$m range of FIR spectrum. Although Planck thermal radiation falls in the 6 - 25 $\mu$m spectrum by nature, all prior art has misunderstood and mistreated the FIR-characteristics of the blackbody thermal radiation. Because, the blackbody thermal radiation behaves like FIR-photons only when it encounters free molecules in gas state, such as water vapors ($H_2O$) and carbon dioxide ($CO_2$) in the atmosphere. Otherwise, it is simply a heat radiation for radiative heat transfer, when it interacts with the atoms or molecules in condensed matters such as solids or liquids.

By contrast, the present invention discloses a ceramic composite capable of emitting not only conventional blackbody thermal radiation but also nonthermal FIR-photon radiation, thanks to the innovative FIR-luminescence mechanism called "persistent phonons-activated FIR-photon emission". The building block of the FIR-luminescence center is $[CrO_6]^{3+}$, or the like.

The ceramic composite of present invention comprises a solid solution, in which the solvent consists of at least 10 wt.% of aluminum oxide and the solute consists of at least 3 wt.% chromium oxide or iron oxide, or both. The solution is purposefully sintered under such a predetermined condition that the aluminum oxides would construct a host lattice with octahedral crystal structure and a part of aluminum ions ($Al^{3+}$) in the octahedral sites would be substituted by chromium ions ($Cr^{3+}$) or iron ions ($Fe^{3+}$) to establish transition meatal ion coordination complexes (TM-ion octahedrons) through counter diffusion of cations during sintering. Furthermore, said ceramic composite is also deliberately processed to cause a slight "Z-in J-T distortion" on the primitive cell of $[CrO_6]^{3+}$ or $[FeO_6]^{3+}$ octahedrons after sintering. A FIR-luminescence center is thus created at each $[CrO_6]^{3+}$ or $[FeO_6]^{3+}$ site.

In this procedure, the degeneracy of orbitals in the $t_{2g}$ group of chromium-ion ($Cr^{3+}$) or iron-ion ($Fe^{3+}$) is removed after sintering, so that the $d_{xy}$ orbital has lower energy than $d_{xz}$ and $d_{yz}$. For the 3 electrons in $3d^3$ configuration of the $Cr^{3+}$ ion, a pair of electrons will occupy the $d_{xy}$ orbital, while the lone electron occupies one of the $d_{xz}$ and $d_{yz}$ orbitals. A comparable scenario may be observed in the case of $[FeO_6]^{3+}$, but with 5 electrons in the $3d^5$ configuration of $Fe^{3+}$ ion.

When said ceramic composite absorbs thermal radiation from the ambience, the incident thermal radiation is converted into phonons (the wave of lattice vibrations). On one hand, the lattice vibrations in the composite will generate heat to increase the body temperature and emit thermal radiation in order to reach and maintain thermodynamic equilibrium with the surrounding. On the other hand, the lattice vibrations will trigger dynamic J-T distortions in the TM-ion octahedrons, swinging between "Z-in" and "Z-out" J-T distortions. Consequently, photons in the 3 - 16 $\mu$m wavelength range are generated and instantly released, with an energy corresponding to the energy difference $\delta_2$ of crystal field splitting. The lattice-vibrations periods are in the order of $10^{-13}$ sec, while the electronic transitions occur in the order of $10^{-16}$ sec. For that reason, the FIR-photon emissions from the ceramic composite of present invention will be spontaneously and continuously.

Even when the ceramic composite has reached in thermodynamic equilibrium with its environment, the lattice vibrations persist and the phonons-activated photon emissions continue. Therefore, the FIR-photon emissions at 3 - 16 $\mu$m wavelengths from the ceramic composite of present invention will last forever.

As disclosed herein, the key attribute of the persistent FIR-luminescence mechanism of present invention is "phonons-activated dynamic J-T distortions". The Jahn-Teller effect is a geometric distortion of a non-linear molecular system that reduces its symmetry and energy. This distortion is typically observed among octahedral complexes where the two axial bonds can be shorter or longer than those of the equatorial bonds. Nonetheless, the effect can also be observed in tetrahedral compounds.

It is worthwhile noting here that the present invention only focuses on the use of octahedral structure $[MO_6]^{3+}$ that leads to the selections of aluminum oxide as part of the solvent and chromium-(III) oxide and/or iron-(III) oxide as solute. Nevertheless, tetrahedral compounds may also be used either separately to make a ceramic composite similar to the one described by present invention, or jointly with octahedral compounds to make a more stable host lattice.

When tetrahedral structure is considered, the solvent may include silicon oxide or any minerals consisting of aluminosilicate, and the solute can be any of titanium oxide, zirconium oxide, cobalt oxide, or iron-(II) oxide, just to name a few, that shares a tetrahedral crystal structure $[MO_4]^{2+}$. Also, when the solvent consists of both silicon oxide and aluminum oxide, a much more stable layered-structure built with interlocked octahedrons and tetrahedrons may be formed.

As disclosed herein, the science of the inventive "persistent phonons-activated FIR-photon emission" (or in other words, "persistent FIR-luminescence"), by which the ceramic composite of present invention can emit not only blackbody thermal radiation but also nonthermal FIR-photon radiation at 3 - 16 $\mu$m wavelengths, is never taught by prior art.

Objects and Advantages

Accordingly, one object of this invention is to provide a ceramic composite that emits not only blackbody thermal radiation but also nonthermal FIR-photon radiation in the 3 - 16 $\mu$m range of wavelength spectrum;

Another object of this invention is to provide a ceramic composite that emits FIR radiation with an effective emissivity greater than 1.0;

Another object of the present invention is to provide a ceramic composite for assembly into a therapeutic device that improves the reaction rate of related biochemical reactions within the treated body for positive biological effects;

Also, another object of the present invention is to provide a simple, easy-to-use, and maintenance-free therapeutic device that is flexible to attach to any part of human or animal body that requires treatment.

These objectives are achieved by a ceramic composite that simultaneously emits blackbody thermal radiation and stimulated FIR-photon radiation in the 3 - 16 $\mu$m wavelength spectrum, with an effective emissivity greater than that expected from a regular blackbody-radiating material. Said ceramic composite comprises a mixture of powders for forming a solid solution, that consists of at least 10 wt.% of aluminum oxide in the solvent and at least 3 wt.% of chromium oxide and/or iron oxide in the solute. Said solution will be sintered to produce a ceramic composite that incorporates a persistent FIR-luminescence mechanism. The ceramic composite may be secured in a flexible attachment means and placed at close proximity of the body part that requires treatment.

Other objects, features, and advantages of the present invention will hereinafter become apparent to those skilled in the art from the following description.

## SUMMARY OF THE INVENTION

In accordance with the present invention, a ceramic composite comprises a solid solution, in which the solvent consists of at least 10 wt.% of aluminum oxide and the solute consists of at least 3 wt.% chromium oxide and/or iron oxide, said solution being sintered into a shaped ceramic article that contains octahedral crystalline structures and features a "persistent phonons-activated FIR-photon emission" mechanism, and is capable of simultaneously emitting the blackbody-like thermal radiation in 4 - 1,000 $\mu$m wavelength spectrum as well as the stimulated FIR-photon radiation in 3 - 16 $\mu$m wavelength band, resulting in an effective emissivity greater than 1.0. The ceramic composite may be used for various purposes, including providing an effective means to improve health conditions of a human or animal body, based on the absorption of 3 - 16 $\mu$m wavelength FIR-photons by the molecules in the body that can enhance chemical reaction rates and thus lead to positive biological effects.

## BRIEF DESCRIPTION OF THE DRAWINGS

FIG. 1 is a perspective view of a first embodiment of the present invention showing a ceramic composite in the shape of a sphere.

FIG. 2 is a perspective view of a second embodiment of the present invention showing a ceramic composite in the shape of a circular plate.

FIG. 3 is a perspective view of a third embodiment of the present invention showing a ceramic composite in the shape of a rectangular plate.

FIG. 4 is a perspective view of a fourth embodiment of the present invention showing a ceramic composite in the shape of a partial cylinder.

FIG. 5 is a top perspective view of a fifth embodiment of the present invention showing multiple ceramic composites are mounted on a flexible substrate for attaching to a body part to be treated, wherein each of ceramic composite has a concave surface facing the body.

FIG. 6 is a bottom perspective view of the embodiment of FIG. 5, showing the pockets containing the concave ceramic composites.

FIG. 7 is a perspective view of a seventh embodiment of the invention, showing an array of IR-emitting elements embedded within a substrate and disposed within an attachment means.

FIG. 8 is a perspective view of an eighth embodiment of the invention, showing an array of IR-emitting elements embedded within a substrate and disposed within an attachment means.

FIG. 9 is a perspective view of an eighth embodiment of the invention, showing an array of IR-emitting elements embedded within a substrate and disposed within an attachment means.

FIG. 10 is a perspective view of an eighth embodiment of the invention, showing an array of IR-emitting elements embedded within a substrate and disposed within an attachment means.

FIG. 11 is a perspective view of an eighth embodiment of the invention, showing an array of IR-emitting elements embedded within a substrate and disposed within an attachment means.

## Reference Numerals in Drawings

| 11 | ceramic composite | 21, 23, 31, 41, 51, 61 | substrate |
|---|---|---|---|
| 22, 28, 34, 44, 54, 64 | pockets | 24, 32, 42, 52, 62 | encasement |
| 25, 30, 40, 50 | straps | 28, 34, 44, 54, 64 | pockets |
| 29, 36, 46, 56, 66 | holes | | |

## DETAILED DESRIPTION OF THE INVENTION

The invention is a ceramic composite that can emit not only the natural blackbody thermal radiation in 4 - 1,000 $\mu$m wavelength spectrum but also the innovative nonthermal FIR-photon radiation in the specified 3 - 16 $\mu$m wavelength band. The ceramic composite features an inventive "persistent phonons-activated FIR-photon emission mechanism" that absorbs ambient thermal radiation to stimulate emissions of FIR-photons in the 3 - 16 $\mu$m wavelength range persistently.

The persistent phonons-activated FIR-photon emission mechanism (or "persistent FIR-luminescence" mechanism) embraces numerous FIR-luminescence centers that are founded on transition-metal (TM) ion coordination complexes with either octahedral or tetrahedral structures. The present invention will only focus on octahedral structures for the sake of easier design, higher efficiency, and more predictable results. A similar composite may be fabricated with tetrahedral structures by following the teachings of present invention, which will be pointed out later.

The building blocks of FIR-luminescence centers are $[CrO_6]^{3+}$, $[FeO_6]^{3+}$, or the like, which consists of a transition metal ion, such as chromium-III ($Cr^{3+}$) or iron-III ($Fe^{3+}$), surrounded by six oxygen anions, forming an octahedral coordination complex. By design, the TM-ion is placed in a weak electrostatic crystal field formed by the anions (or oxoanions) in the host lattice together with the surrounding doped cations. In this arrangement, the energy $\delta_2$ of crystal field splitting can be devised by prearranged type and number of doped cations to be around 100 - 315 meV, based on CFT modeling. This corresponds to a wavelength spectrum in 4 - 12 $\mu$m, with a projected photoluminescence profile covering the specified 3 - 16 $\mu$m wavelength range.

Accordingly, the ceramic composite comprises a solid solution, with the amount of solvent > 50 wt.% and solute < 50 wt.%. Said solvent consists of at least 10 wt.% aluminum oxide ($Al_2O_3$) that helps construct the required octahedral crystalline framework in host lattice for the persistent FIR-photon emission mechanism. The rest wt.% of solvent may include silicon oxide ($SiO_2$) for building a more stable host lattice that contains aluminosilicate ($Al_2SiO_5$). Besides, its oxoanions ($SiO_4^{4-}$, $AlSiO_4^-$) also provide a viable approach for fine-tuning the crystal field splitting.

In implementation, the solvent may make up to 50 - 80 wt.% of the solution and consists of about 10 - 40 wt.% $Al_2O_3$ and 10 - 40 wt.% $SiO_2$. To give an example, 20 wt.% aluminum oxide and 40 wt.% silicon oxide may be used to build a stable host lattice that holds a layered structure with interlocked tetrahedrons and octahedrons.

The present invention uses Cr and Fe respectively in separate cases for substituting Al in the host lattice to demonstrate the concept. The transition metal ions, $Cr^{3+}$ or $Fe^{3+}$, are expected to replace $Al^{3+}$ ions in the octahedral sites of host lattice during sintering.

Typically, the solute may contain two parts. The first part, mainly for constructing the FIR-luminescence centers, shall comprise at least 3 wt.% chromium oxide ($Cr_2O_3$) and/or iron oxide ($Fe_2O_3$). The quantity may be in the range of 3 - 40 wt.%, though 10 - 20 wt.% is preferred.

The second part of solute, for fine-tuning the crystal field splitting, may include 3-20 wt.% oxides of other transition metals such as zirconium (Zr), titanium (Ti), manganese (Mn), iron (Fe), cobalt (Co), nickel (Ni), copper (Cu), zinc (Zn), or niobium (Nb), and alkali or alkaline earth metals such as lithium (Li), sodium (Na), potassium (K), magnesium (Mg), or Calcium (Ca). The cations from transition metal or metal elements are utilized to manipulate the electrostatic crystal field, by interstitially fitting into the space between solvent particles in the host lattice during sintering.

It is worthwhile noting here that among aforesaid transition metals, the elements Zr, Ti, Co, and Ni may be managed to substitute Si in the $[SiO_4]^{4-}$ tetrahedral structures of the host lattice, under related sintering conditions. In this case, they may act as additional FIR-luminescence centers. Or, themselves may be used alone to produce a ceramic composite similar to the one disclosed in present invention. It will be obvious to anyone skilled in the art to make such a composite by following the teachings of present invention, but simply changing the process parameters associated with the selected materials.

In one embodiment, the solid solution of present invention includes 70 wt.% of solvent and 30 wt.% solute. The solvent consists of 45 wt.% silicon oxide and 25 wt.% aluminum oxide. The solute consists of 25 wt.% transition metal oxides, including 5% chromium oxide and 15% iron oxide for FIR-emitting purpose, and with the rest 5 wt.% of minor transition oxides, such as zirconium oxide, titanium oxide, and zinc oxide, as cations-dopants. The second part of solute consist of up to 5 wt.% in total calcium oxide, potassium oxide and magnesium oxide, for cation-doping.

After appropriate oxides being selected, the mixture of these oxides can be processed into a shaped article or powders form for practical uses, such as in the therapeutic devices. The process involves mixing all constituent oxides with bonding agents and stabilizers, followed by grinding, drying, forming, green machining, and sintering. The consequent FIR radiation spectrum and its spectral strength depend on the resultant crystalline structure, which may be influenced by many factors, including sintering temperature, length of sintering time, and a course with specified steps and correlated heating/cooling rates.

The powder mixture of the solid solution system will be heated to a temperature that is in the range of 0.5 - 0.75 of the melting temperature. For $Al_2O_3$ with a melting temperature of 2,073 °C, the sintering temperature is commonly in about 1,000 - 1,600 °C. During the solid-state sintering process, the powder does not melt. Instead, the joining together of the particles and the reduction in the porosity (densification) of the body occur by atomic diffusion in the solid states.

The simplest system involves the reaction between two solid phases, *A* and *B,* to produce a product C. For example, when both aluminum oxide and silicon oxide are used as solvent, the formation of the product, aluminosilicate ($Al_2SiO_5$) for the host lattice, at a temperature above 1,100 °C can be presented as:

$$Al_2O_3 + SiO_2 \rightarrow Al_2SiO_5$$

For the meantime during the course of sintering, chromium cations ($Cr^{3+}$) or iron cations ($Fe^{3+}$) may take over

the octahedral sites of aluminum cations ($Al^{3+}$) in aluminosilicate lattice and form FIR-luminescence centers, denoted by [$Al_2SiO_5$:$Cr^{3+}$] or [$Al_2SiO_5$:$Fe^{3+}$], respectively.

The reaction mechanism involves counter diffusion of the cations ($Al^{3+}$ vs. $Cr^{3+}$ or $Fe^{3+}$). The cations migrate in opposite directions with the oxygen ions remaining essentially stationary. This mechanism is the most likely mechanism, in which the cation flux is coupled to maintain electroneutrality.

When the rate of product formation is controlled by diffusion through the product C layer, the product thickness $y$ is observed to follow a parabolic growth law:

$$y^2 = Kt$$

where $t$ is the length of sintering time and $K$ is the rate constant that obeys the Arrhenius relation.

The reaction is said to be 100% (complete), when $y = r_a + r_b$, $r_a$ and $r_b$ being the radius of reactants $A$ and $B$, respectively. It means that the reaction is diffusion controlled and in practice, the diffusion coefficients of such cations differ widely.

The reaction rate (A) will decrease with temperature according to the Arrhenius relation. The sintering time required for completing the reaction increases with an increase in the particle size of reactants ($r_a$, $r_b$) because the diffusion distance ($r_a + r_b$) will increase. Thus, the powder characteristics of greatest interest would be the size, size distribution, shape, degree of agglomeration, chemical composition, and purity.

Generally, larger particles (> 10 $\mu$m) need a higher temperature and longer time to react because of the diffusion mechanism during sintering. A continuing trend is toward the preparation of fine powders, in particular very fine powders with a particle size in 50 - 100 nm (often referred to as nanoscale powders). However, as the size decreases below 1 $\mu$m, the particles exhibit a greater tendency to interact, leading to the formation of agglomerates so that it may require proper control.

Besides, the kinetics of grain growth is also influenced by the grain size distribution. If the distribution is large, the pressure difference between the smaller and larger grains is very high, and thus the growth of larger grains at the expense of smaller ones is much faster than where the distribution is narrow. So, a narrow distribution of powder sizes, for examples, 50 - 100 nm for nano-powders or 1 - 10 $\mu$m for micron-particles is preferred. Nonetheless, the optimal particle size and size distribution for practical implementation would be 0.1 - 1 $\mu$m.

Normally, the homogeneity of mixing is one of the most critical parameters. It influences the diffusion distance between reactants and the relative number of contacts between the reactant particles, and thus to produce a homogeneous product. Incomplete reactions, especially in poorly mixed powders, produce undesirable phases.

Higher temperature usually increases the rate of sintering mechanisms and reduces the time required for accomplishing the diffusion reactions. Higher temperature can accelerate volume diffusion, compared to interfacial diffusion. Since grain growth is often controlled by surface diffusion, while densification is controlled by volume diffusion or grain boundary diffusion, higher temperature often leads to higher densification, compared to grain growth. Given that interfacial diffusion mechanisms are preferred to volume diffusion, it is necessary to choose an optimal sintering temperature. Additionally, to allow proper phase transformation of the participating elements into the designed polycrystalline structure, a heating cycle that is divided into several steps with correlated heating/cooling rate is required.

That said, in at least one embodiment of the present invention, a course of three-steps, with a heating rate of 5 - 10 °C/min to reach a temperature at 1,200 °C and sintering the solution for at least 2 hours, is used to allow necessary phase transformations. Afterwards, a controlled cooling is also needed to secure the phases in the designed polycrystalline structure.

One key aspect in present invention is to create FIR-luminescence centers with octahedral complexes of [$CrO_6$]$^{3+}$, [$FeO_6$]$^{3+}$, or the like. And, the other aspect is to generate an appropriate electrostatic crystal field that surrounds the complex. If everything goes well with the design, the resultant ceramic composite will be capable of emitting nonthermal FIR-photon radiation in the specific 3-16 $\mu$m wavelength band. Said ceramic composite can be explicitly demonstrated to have an effective emissivity greater than 1.0 by using a commercially available sensor in the 8 - 14 $\mu$m wavelength range.

Despite above guidelines, the solid-state reaction in powder systems depends on several parameters, such as the chemical nature of the reactants and the product, the size, the size distribution and shape of the particles, the relative sizes of the reactant particles in the mixture, the uniformity of the mixing, the reaction atmosphere, the temperature, the heating/cooling rate and the time, and so forth. Thus, the result is difficult to predict with a simplified theoretical modeling.

In practice, numerous experiments have to be performed in order to observe the influence of synthesis parameters on polycrystalline structure and material properties. Various experimental samples had been tried out with different sintering temperatures and heating courses before a synthesis process of the ceramic composite of present invention could be finalized. The fabrication process is surely nontrivial. Yet, any one skilled in ceramic processing should be able to figure out his own synthesis parameters experimentally by following the teaching described herein.

With carefully chosen composition and synthesis parameters, the subsequent ceramic composite would produce a blend of crystalline and amorphous regions. Each crystalline region allows electron transitions within the $[CrO_6]^{3+}$or $[FeO_6]^{3+}$ octahedral structures that functions as a dipole, governed by the inventive persistent phonons-activated FIR-photon emission mechanism. The stimulated FIR-photons in the 3 - 16 $\mu$m wavelength spectrum are so generated.

The ceramic composite of present invention, prepared as disclosed above, has an overall FIR radiation, including both the blackbody-like thermal radiation and the stimulated FIR-photon radiation, that can be approximated as a black-body radiation at a temperature at least 1 °K (or 1 °C) higher than the actual body temperature of said ceramic composite. It signifies an effective emissivity greater than 1.0 ($\varepsilon > 1.0$).

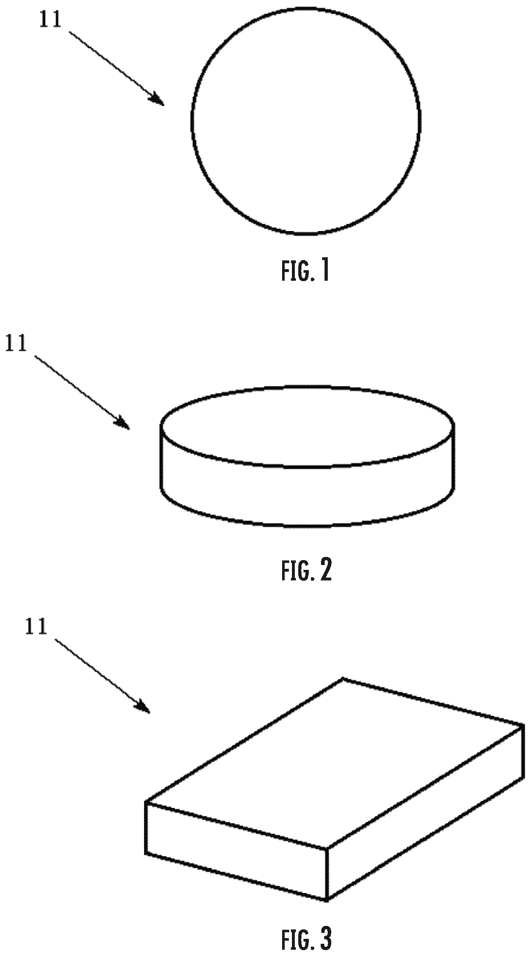

FIGS. 1-3 show three separate embodiments of the present invention of varying shapes: in FIG. 1, the ceramic composite 11 is shaped as a sphere, in FIG. 2, the ceramic composite 11 is shaped as a circular plate, and in FIG. 3, the ceramic composite 11 is shaped as a rectangular plate. It can also be prepared in form of powders, though the bulk-form has at least three times higher radiation efficiency than the powder-form with the same mass.

The ceramic composite(s) 11 of the present invention may be formed into various shapes and sizes, depending upon the particular applications. In at least one embodiment, the ceramic composites may be circular in shape, and may be a 2 - 50 mm diameter circle with a thickness of 1-10 mm. In another embodiment, the ceramic composites may be rectangular, having dimensions of a 2 by 3 mm rectangle to a 40 by 50 mm rectangle, with a thickness of 1 - 10 mm. Rectangular and circular shaped ceramics are generally easier to fabricate than other shapes.

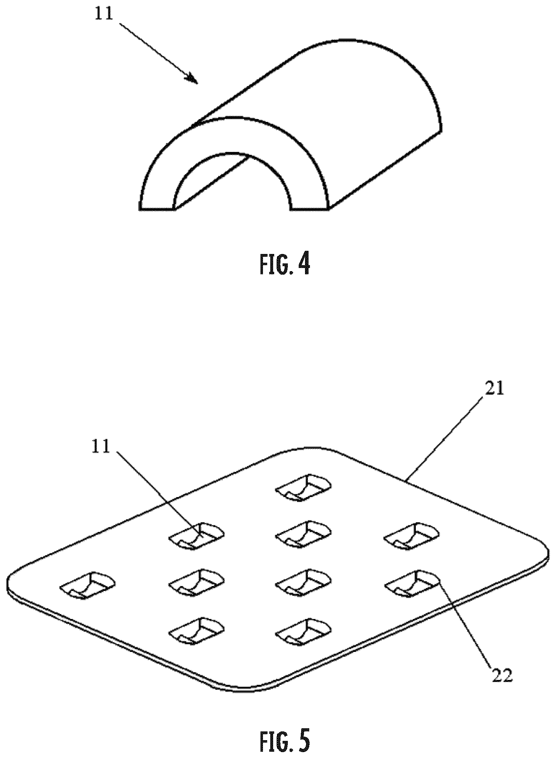

Nonetheless, it may be advantageous to form the ceramic composite 11 with a concave shape. It is anticipated that a concave surface will help focus the rays of radiation emitted by the ceramic composite in a region or point at a distance from the surface of the ceramic composite. The concave surface may take a variety of shapes, such as hemispherical, bowl-shaped, or a partial cylinder. FIG. 4 shows an embodiment of the present invention, in which ceramic composite 11 has a partial cylinder shape.

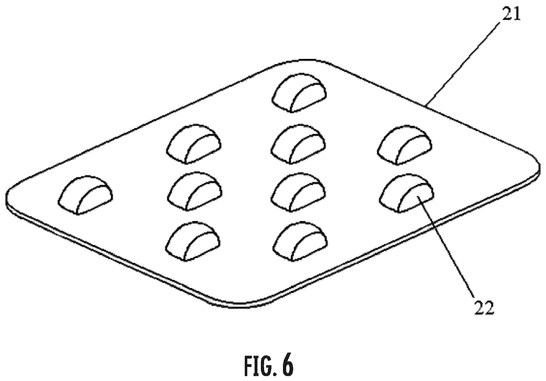

FIG. 5 shows a preferred embodiment of the present invention for therapeutic applications, in which multiple ceramic composites 11 are embedded in a substrate 21, which may be made from silicone, or similar materials. The substrate 21 is a substantially flat sheet that includes a number of pockets 22, which are curved protrusions dimensioned to contain ceramic composites 11. FIG. 6 illustrates the underside of substrate 21, which faces away from the body part being treated. In this embodiment, all pockets 22 have the same dimension because all ceramic composites 11 have the same dimensions. In other embodiments, however, pockets 22 may have different sizes or shapes tailored to specific applications or arrangements of variable ceramic composites 11.

Also, the ceramic composite 11 in FIG. 5 has a partial cylindrical shape. The partial-cylindrical shaped ceramic composite is arranged to have the concave surface facing toward the body part to be treated. This arrangement helps to focus FIR radiation at about one (1) inch above the surface of the device. When the device is wrapped closely around the body during use, the radiation will be focused to a depth of about one inch into the body tissue, and thus significantly enhance the radiation effect in the body.

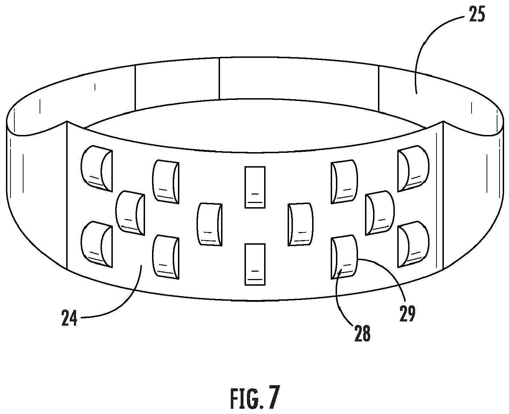

FIG. 7 shows another embodiment of the invention in which a substrate 23 is enclosed within an attachment means. Preferably, the attachment means comprises an encasement 24 and straps 25 attached to both ends of the encasement 24. In the embodiment shown in FIG. 7, the encasement 24 includes holes 29 formed in one side. The holes 29 are dimensioned and positioned to allow pockets 28 of the substrate 23 to partially protrude outside the encasement 24 such that the encasement 24 is substantially flat on the side facing the user. However, other configurations are also within the scope of the invention, such as encasements lacking holes. This embodiment may be adapted to fit around a user's waist, such that the pockets 28 generally target FIR radiation around the user's lower back.

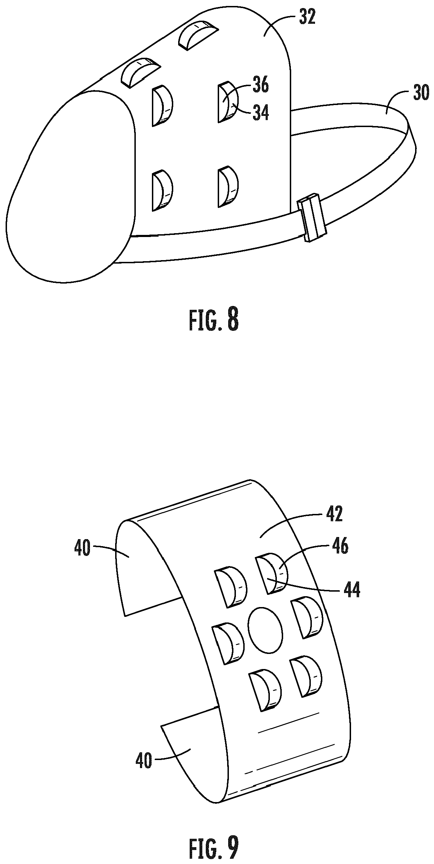

FIG. 8 shows another embodiment of the invention in which a substrate 31 is enclosed within an attachment means. Preferably, the attachment means comprises an encasement 32 and straps 30 attached to both ends of the encasement 32. In the embodiment shown in FIG. 8, the encasement 32 includes holes 36 formed in one side. The holes 36 are dimensioned and positioned to allow pockets 34 of the substrate 31 to partially protrude outside the encasement 32 such that the encasement 32 is substantially flat on against the user's body. However, other configurations are also within the scope of the invention, such as encasements lacking holes. This embodiment may be adapted to fit around a user's shoulder, such that the pockets 34 generally target FIR radiation around the user's shoulder.

FIG. 9 shows another embodiment of the invention in which a substrate 41 is enclosed within an attachment means. Preferably, the attachment means comprises an encasement 42 and straps 40 attached to both ends of the encasement 42. In the embodiment shown in FIG. 9, the encasement 42 includes holes 46 formed in one side. The holes 46 are dimensioned and positioned to allow pockets 44 of the substrate 41 to partially protrude outside the encasement 42 such that the encasement 42 is substantially flat on the side facing the user. However, other configurations are also within the scope of the invention, such as encasements lacking holes. This embodiment may be adapted to fit around a user's knee, such that the pockets 44 generally target FIR radiation around the user's knee cap.

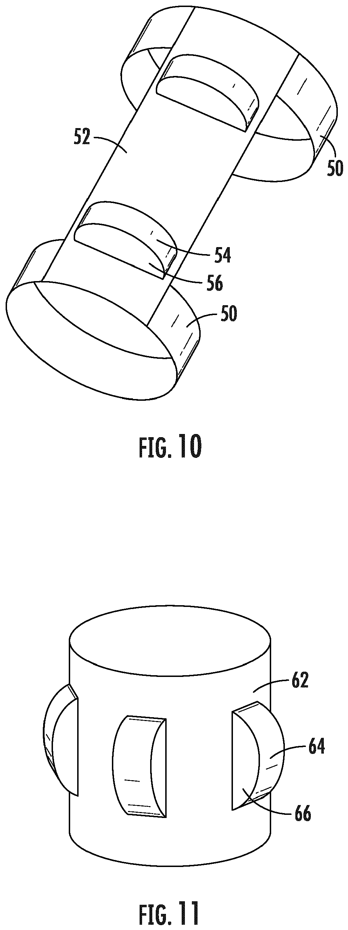

FIG. 10 shows another embodiment of the invention in which a substrate 51 is enclosed within an attachment means. Preferably, the attachment means comprises an encasement 52 and straps 50 attached to both ends of the encasement 52. In the embodiment shown in FIG. 10, the encasement 52 includes holes 56 formed in one side. The holes 56 are dimensioned and positioned to allow pockets 54 of the substrate 21 to partially protrude outside the encasement 52 such that the encasement 52 is substantially flat on the side facing the user. However, other configurations are also within the scope of the invention, such as encasements lacking holes. This embodiment may be adapted to fit around a user's wrist, such that the pockets 54 generally target FIR radiation around the user's wrist.

FIG. 11 shows another embodiment of the invention in which a substrate 61 is enclosed within an attachment means. Preferably, the attachment means comprises an encasement 62 that can separate to be installed around a user's body part. In the embodiment shown in FIG. 11, the encasement 62 includes holes 66 formed in one side. The holes 66 are dimensioned and positioned to allow pockets 64 of the substrate 21 to partially protrude outside the encasement 62 such that the encasement 62 is substantially flat on the side facing the user and the encasement 62 wraps around a user's body part, such as a forearm, ankle, or shin, among others. However, other configurations are also within the scope of the invention, such as encasements lacking holes. This embodiment may be adapted to fit around a user's wrist, such that the pockets 64 generally target FIR radiation around the user's body part.

Substrates 21, 23, 31, 41, 51, and 61 in various embodiments may be made from silicone (polydimethylsiloxane), zinc sulfide, sodium chloride, potassium bromide, or similar material.

In experiments, the ceramic composites are made into a shape of 1/3-circumference cutout of a 12-mm long cylindrical tube, with 10-mm I.D. (inner diameter) and 20-mm O.D. (outer diameter). The specific spectral luminance of the ceramic composite was measured to cover the 3 - 16 $\mu$m wavelength spectrum. Furthermore, the thermal radiation that adds up blackbody-like thermal radiation to the stimulated FIR-photon radiation in the 8 - 14 $\mu$m wavelength range was measured by a commercial sensor and approximated as blackbody radiation at a temperature about 3 °C higher than the actual temperature of said ceramic composite, which signifies an effective emissivity of 1.04 ($\varepsilon$ = 1.04).

### Conclusion, Ramifications, and Scope

According to the present invention, a ceramic composite features a persistent phonons-activated FIR-photon emission mechanism that is capable of simultaneously emitting blackbody thermal radiation in 4 - 1,000 $\mu$m wavelength spectrum as well as the stimulated FIR-photon radiation in the 3 - 16 $\mu$m wavelength band, resulting in an effective emissivity greater than 1.0. The ceramic composite may be used for various purposes, including providing an effective means to improve health conditions of a human or animal body, based on enhanced chemical reaction rates in the body by absorption of 3 - 16 $\mu$m wavelength FIR-photons that leads to positive biological effects.

The invention has been described above. Obviously, numerous modifications and variations of the present invention are possible in light of the above teachings. Such variations are not to be regarded as a departure from the spirit and scope of the invention and all such modifications as would be obvious to one skilled in the art are intended to be included within the scope of the following claims.

### Claims

1.  A method of producing a non-thermal far infrared radiation (FIR) emitting ceramic composite comprising:

    a) heating a powdered material at a heating rate to reach a sintering temperature;
    b) sintering said powdered material at the sintering temperature for a sintering time;
    c) cooling said powdered material at a cooling rate to form a ceramic composite; wherein steps a) through c) produce at least one phase transformation such that the ceramic composite comprises at least one far infrared luminescence center of at least one octahedral complex formed by a transition metal ion surrounded by six oxygen anions or one tetrahedral complex formed by a transition metal ion surrounded by four oxygen anions; and

    wherein said anions generate an electrostatic crystal field around said complexes; and resulting in said ceramic composite emitting non-thermal far infrared radiation in the 3 - 16 $\mu$m wavelength spectrum, wherein the overall measurable radiation over the 8 - 14 $\mu$m wavelength range is approximated as blackbody radiation at a temperature at least 1 °C higher than the actual body temperature of said ceramic composite, signifying an effective emissivity greater than 1.0.

2.  A method of claim 1:

    wherein said material is created by mixing a solvent of about 10% or more by weight of a first oxide; and
    a solute comprising about 3% or more by weight of a second oxide and about 3% to about 20% by weight of a third oxide.

3.  A method of claim 1, wherein:

the first oxide comprises one or more of the following: silicon oxide and aluminum oxide or wherein:
the second oxide comprises one or more of the following: chromium oxide and iron oxide.

4. The method of claim 1, wherein:
the third oxide comprises one or more of the following: zirconium oxide, titanium oxide, manganese oxide, iron oxide, cobalt oxide, nickel oxide, copper oxide, zinc oxide, niobium oxide, lithium oxide, sodium oxide, potassium oxide, magnesium oxide, and calcium oxide.

5. The method of claim 1, wherein:
the heating rate is between about 5 degrees and about 10 degrees Celsius per minute.

6. The method of claim 1, wherein:

the sintering temperature is between about 1,000 and 1,600 degrees Celsius or wherein:
the sintering temperature is between about 50% and about 75% of the melting temperature of the solvent.

7. The method of claim 1, wherein:
the sintering time is at least two hours.

8. A composition of a non-thermal far infrared radiation emitting ceramic composite comprising:

a solvent of about 10% or more by weight of a first oxide; and
a solute comprising about 3% or more by weight of a second oxide and about 3% to about 20% by weight of a third oxide.

9. The composition of claim 8, wherein:

the first oxide comprises one or more of the following: silicon oxide and aluminum oxide or wherein:
the second oxide comprises one or more of the following: chromium oxide and iron oxide.

10. The composition of claim 8, wherein:
the third oxide comprises one or more of the following: zirconium oxide, titanium oxide, manganese oxide, iron oxide, cobalt oxide, nickel oxide, copper oxide, zinc oxide, niobium oxide, lithium oxide, sodium oxide, potassium oxide, magnesium oxide, and calcium oxide.

11. A therapeutic device for treating a human or animal body comprising:
a ceramic module capable of emitting blackbody thermal radiation and stimulated far infrared photon radiation with an effective emissivity greater than about 1.0 affixed with a flexible means for attaching the module to a body part to be treated.

12. The therapeutic device of claim 11, wherein:
the ceramic module emits blackbody thermal radiation in wavelengths of about 4 to about 1,000 $\mu$m and stimulated FIR-photon radiation for wavelengths between about 3 to about 16 $\mu$m.

13. The therapeutic device of claim 11, wherein:
the ceramic module comprises three oxides.

14. The therapeutic device of claim 13, wherein:

a first oxide comprises one or more of the following: silicon oxide and aluminum oxide or wherein:
a second oxide comprises one or more of the following: chromium oxide and iron oxide or wherein:
a third oxide comprises one or more of the following: zirconium oxide, titanium oxide, manganese oxide, iron oxide, cobalt oxide, nickel oxide, copper oxide, zinc oxide, niobium oxide, lithium oxide, sodium oxide, potassium oxide, magnesium oxide, and calcium oxide.

15. The therapeutic device of claim 11, wherein:

the ceramic module includes at least one FIR luminescence center of at least one octahedral complex formed by a transition metal ion surrounded by six oxygen anions, said anions generating an electrostatic crystal field around said complexes; and resulting in said ceramic composite having a persistent phonons-activated FIR-photon emission mechanism or wherein:

the ceramic module includes at least one FIR luminescence center of at least one tetrahedral complex formed by a transition metal ion surrounded by four oxygen anions, said anions generating an electrostatic crystal field around said complexes; and resulting in said ceramic composite having a persistent phonons-activated FIR-photon emission mechanism.

11

FIG. 1

11

FIG. 2

11

FIG. 3

11

FIG. 4

21

11

22

FIG. 5

FIG. 6

FIG. 7

FIG. 8

FIG. 9

FIG. 10

FIG. 11

Europäisches
Patentamt

European
Patent Office

Office européen
des brevets

# EUROPEAN SEARCH REPORT

**Application Number**

EP 24 17 5893

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/080964 A1 (WEY ALBERT CHIN-TANG [US]) 16 March 2023 (2023-03-16) * paragraphs [0012] - [0014], [0087] - [0089], [0096]; claims 1-14 * | 1-15 | INV. C04B35/18 A61N5/06 C04B35/626 C04B35/64 |
| A | US 2021/228903 A1 (WEY ALBERT CHIN-TANG [US]) 29 July 2021 (2021-07-29) * claims 1-20 * | 1-15 | |
| A | JIE LIU ET AL: "Strengthening mechanism of far-infrared radiation of tourmaline in iron-tailing ceramics", CERAMICS INTERNATIONAL, ELSEVIER, AMSTERDAM, NL, vol. 47, no. 18, 30 January 2021 (2021-01-30), pages 25214-25220, XP086713083, ISSN: 0272-8842, DOI: 10.1016/J.CERAMINT.2021.01.278 [retrieved on 2021-01-30] * Section 2.3: Characterization * | 1-15 | |

**TECHNICAL FIELDS SEARCHED (IPC)**

C04B
A61N

The present search report has been drawn up for all claims

| Place of search | Date of completion of the search | Examiner |
|---|---|---|
| The Hague | 25 September 2024 | Süzük, Kerem Güney |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

Europäisches
Patentamt

European
Patent Office

Office européen
des brevets

**Application Number**

EP 24 17 5893

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

    1. claims: 1-15

            A non-thermal far infrared radiation emitting ceramic

    1.1. claims: 1-7

            A method of producing a non-thermal far infrared radiation emitting ceramic composite

    1.2. claims: 8-10

            A composition of a non-thermal far infrared radiation emitting ceramic composite

    1.3. claims: 11-15

            A therapeutic device
                        - - -

Please note that all inventions mentioned under item 1, although not necessarily linked by a common inventive concept, could be searched without effort justifying an additional fee.

## ANNEX TO THE EUROPEAN SEARCH REPORT
## ON EUROPEAN PATENT APPLICATION NO.

EP 24 17 5893

This annex lists the patent family members relating to the patent documents cited in  the above-mentioned European search report.
The members are as contained in the European Patent Office EDP file on
The European Patent Office is in no way liable for these particulars which are merely given for the purpose of information.

25-09-2024

| Patent document cited in search report | Publication date | Patent family member(s) | | Publication date |
|---|---|---|---|---|
| US 2023080964 A1 | 16-03-2023 | CA | 3169853 A1 | 13-03-2023 |
| | | CN | 115806437 A | 17-03-2023 |
| | | EP | 4147748 A2 | 15-03-2023 |
| | | JP | 2023055638 A | 18-04-2023 |
| | | US | 2023080964 A1 | 16-03-2023 |
| US 2021228903 A1 | 29-07-2021 | CA | 3104400 A1 | 24-07-2021 |
| | | EP | 3854452 A1 | 28-07-2021 |
| | | US | 2021228903 A1 | 29-07-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 17473799 A
- US 8285391 B
- US 9308388 B
- US 9962441 B
- US 10610699 B
- US 20210228903 A
- US 473799